(19) Europäisches Patentamt
European Patent Office
Office européen des brevets

(11) Veröffentlichungsnummer: **0 273 407 B1**

(12) **EUROPÄISCHE PATENTSCHRIFT**

(45) Veröffentlichungstag der Patentschrift: **24.08.94**

(51) Int. Cl.5: **A61K 35/78**, A61K 35/60, A61K 31/355, //(A61K35/78, 35:60,31:355,31:23)

(21) Anmeldenummer: **87119188.8**

(22) Anmeldetag: **24.12.87**

Die Akte enthält technische Angaben, die nach dem Eingang der Anmeldung eingereicht wurden und die nicht in dieser Patentschrift enthalten sind.

(54) **Mittel zur Verbesserung spezifischer Eigenschaften des Blutes.**

(30) Priorität: **30.12.86 DE 3644677**

(43) Veröffentlichungstag der Anmeldung:
**06.07.88 Patentblatt  88/27**

(45) Bekanntmachung des Hinweises auf die Patenterteilung:
**24.08.94 Patentblatt  94/34**

(84) Benannte Vertragsstaaten:
**AT BE CH DE FR GB IT LI LU NL SE**

(56) Entgegenhaltungen:
**EP-A- 0 152 106**
**EP-A- 0 204 987**

**PATENT ABSTRACTS OF JAPAN, Band 10, Nr. 239 (C-367)(2295), 19. August 1986; &JP-A-61 72717 (KAO CORP.) 14-04-1986**

**L. BEZANGER-BEAUOUESNE et al.: "Plantes médicinales des régions tempérées",1980, Seiten 50-51, Maloine S.A. Herausgeber,Paris, FR**

(73) Patentinhaber: **Ismail, Roshdy, Dr.**
**Siebengebirgs-Apotheke**
**Siebengebirgsallee 2**
**D-50939 Köln (DE)**

(72) Erfinder: **Ismail, Roshdy, Dr.**
**Siebengebirgs-Apotheke**
**Siebengebirgsallee 2**
**D-50939 Köln (DE)**

(74) Vertreter: **Werner, Hans-Karsten, Dr. et al**
**Patentanwälte**
**Von Kreisler-Selting-Werner**
**Postfach 10 22 41**
**D-50462 Köln (DE)**

THE MERCK INDEX, 10TH EDITION, 1983, NO. 5271 "LECITHIN"

THE MERCK INDEX, 10TH EDITION, 1983, NO. 8574 "SOYBEAN OIL"

The Merck Index, 10th Edition, 1983, no. 5271, "Lecithin"

The Merck Index, 10th Edition, 1983, no. 8574 "Soybean Oil"

**Beschreibung**

Die Erfindung betrifft Zusammensetzungen nach Anspruch 1 sowie Verwendungen der Zusammensetzung.

Der Einfluß von Vitamin E bzw. Tocopherol und seinen Derivaten auf charakteristische Eigenschaften des Blutes ist seit längerer Zeit bekannt. Im Gegensatz zu immer wieder auftretenden Vermutungen, daß Vitamin E auch schon in kleinen Dosen eine signifikante physiologische Wirksamkeit entfalte, ist es inzwischen wissenschaftlich erwiesen, daß Vitamin E eine signifikante pharmakologische Wirkung erst dann zeigt, wenn es in Tagesdosen von über 600 mg verabreicht wird. Die EP-A-0 204 987 des Anmelders beschreibt Mittel zur Verbesserung der Eigenschaften des Blutes, die 400 mg Tocopherol oder Ester des Tocopherols pro Verabreichungseinheit enthalten, wobei mindestens zwei derartige Verabreichungseinheiten pro Tag aufgenommen werden müssen. Das auf diesem Wege zugeführte Vitamin E bewirkt eine deutliche Verbesserung der Erythrozytenflexibilität, wobei in der genannten Druckschrift ein synergistischer Effekt des Vitamin E und der gleichzeitig verabreichten durchblutungsfördernden und/oder gefäßerweiternden Mittel beschrieben wird. Die verbesserten Eigenschaften des Blutes eines Patienten, dem Tagesdosen von 600 bis 800 mg Vitamin E zusammen mit durchblutungsfördernden und/oder gefäßerweiternden Mitteln verabreicht wurden, bewirken eine Stärkung der Immunabwehr, eine Verbesserung der Durchblutung der Peripherie der Augen, des Mittelohrs, des Herzens und des Cerebrums, was auf die günstigen Fließeigenschaften des Blutes zurückgeführt wird.

Zwiebeln und Knoblauch wird schon seit altersher eine gesundheitsfördernde Wirkung zugeschrieben. Schon seit Jahrtausenden sind Knoblauch und Zwiebel Bestandteile der Volksmedizin. Untersuchungen aus jüngerer Zeit zeigen auch, daß die aus Knoblauch und Zwiebeln extrahierten Öle Blutplättchen am Zusammenballen hindern. Dies soll bewirken, daß der Genuß der Knollen gegen Schlaganfall, Thrombose und Arteriosklerose schützt. Dafür werden die Inhaltsstoffe der beiden Knollen verantwortlich gemacht. Außerdem ist es bekannt, daß Knoblauch bei einer Dosierung von 1800 mg und mehr pro Tag die Eigenschaft hat, die Blutlipid-Werte zu senken. Insbesondere wurde eine cholesterin- und triglyceridesenkende Wirkung nachgewiesen. Seit langer Zeit ist es außerdem bekannt und gehört zu den überlieferten Kenntnissen der Volksmedizin, die inzwischen auch wissenschaftlich bestätigt wurden, daß sowohl Knoblauch als auch Zwiebeln Wirkstoffe enthalten, die die Eigenschaften milder Antibiotika haben. Derartige Wirkstoffe wurden mittlerweile als Antiseptika bzw. Bacteriostatika schon in hohen Verdünnungen bestätigt.

Patent Abstracts Japan, Vol. 10, Nr. 239 (C-367) (2295), 1986 beschreibt die periphere Durchblutung verbessernde Mittel, welche ungesättigte Fettsäuren, insbesondere Linol- und Linolensäure bzw. deren Ester enthalten, sowie Antioxidanzien wie d,l-alpha-Tocopherol und andere, um die Wirkung der ungesättigten Fettsäuren zu verbessern.

Die EP 0 152 106 beschreibt Mittel enthaltend Vitamin E, die mit gefäßerweiterenden und/oder durchblutungsfördernden Mitteln kombiniert sind, als Rheumamittel.

L. Bézanger-Beauquesne et al., 1980, 50-51, "Plantes médicinales des régions tempérées" beschreibt Eigenschaften von Allium sativum wie vasodilatatorische Effekte auf die peripheren Gefäße, die positive Auswirkung auf die Thrombozytenaggregation zur Vorbeugung von Thrombosen und Arteriosklerose und weitere Eigenschaften wie antiseptische Natur.

Aus The Merck Index, 10th ed., No. 8574, wird die durchschnittliche Zusammensetzung von Sojabohnenöl offenbart. Daraus ergibt sich, daß dieses Öl ungesättigte und mehrfach ungesättigte Fettsäuren enthält.

Merck Index, 10th ed., No. 5271, weist darauf hin, daß Sojabohnenlecithin ungesättigte Säuren wie Palmitoleic, Oleic, Linoleic, Linolenic acids , $C_{20}$ - $C_{22}$ Säuren inklusive Arachidonsäure enthält.

Überraschend wurde nun gefunden, daß die gemeinsame Verabreichung von Wirkstoffen des Knoblauchs mit Vitamin E zu einer synergistischen Verstärkung der Wirkung der Einzelkomponenten führt. Ebenfalls überraschend sind Ergebnisse, daß auch die in Zwiebeln enthaltenen Wirkstoffe in ihren Eigenschaften durch Vitamin E synergistisch verstärkt werden, wenn dieses in Tagesdosen über 400 mg verabreicht wird.

Die pharmakologische Bedeutung von aus marinen Lebewesen gewonnenen Fetten und Ölen ist ebenfalls seit langer Zeit bekannt. Dies ist nicht nur auf den hohen Gehalt an physiologisch wertvollen, ungesättigten Fettsäuren bzw. Estern derartiger hochungesättigter Fettsäuren zurückzuführen, sondern auch darauf, daß die aus marinen Lebewesen gewonnenen Fette und Öle vergleichsweise große Mengen an fettlöslichen Vitaminen, insbesondere an Vitamin A und Vitamin D, enthalten. Die Verabreichung von Lebertran stellt beispielsweise die Versorgung des kindlichen Körpers mit den genannten Vitaminen und ungesättigten Fettsäuren bzw. Estern derartiger Fettsäuren sicher. Zum Erreichen eines bestimmten therapeutischen Zieles, beispielsweise der ausreichenden Versorgung des Körpers mit den angegebenen Vitaminen, war eine Tagesdosis an Lebertran oder vergleichbaren Fischölen in der Größenordnung von 30 g oder mehr einzuhalten, die unter Umständen auf mehrere Verabreichungseinheiten verteilt werden konnte. Überraschend wurde nun gefunden, daß die Tagesdosis auf eine Menge von 10 bis 15% des oben angegebenen Wertes reduziert werden kann, wenn man Lebertran oder Fischöle enthaltende Zusammensetzungen zusammen mit Vitamin E verabreicht. Es kann eine Mengenreduktion bis zu 40% erreicht werden. Überraschend war es weiterhin, daß auch die Vitamin E-Tagesdosen in derartigen Zusammensetzungen reduziert werden können und dabei die bekannten therapeutischen Wirkungen des Vitamin E synergistisch verstärkt werden.

Die Erfindung betrifft Zusammensetzungen zur Verbesserung der Eigenschaften des Blutes, enthaltend pro Verabreichungseinheit

(a) Vitamin E und/oder eines seiner physiologisch aktiven Derivate in hoher Dosierung von 75 bis 1200 mg,

(b) eine oder mehrere Komponente(n) aus der Gruppe Extrakte oder Konzentrate des Knoblauchs, Knoblauchölmazerate und/oder Knoblauchölextrakte und/oder Knoblauchpulver, Extrakte oder Konzentrate der Zwiebel oder Zwiebelöl und/oder Zwiebelölextrakte und/oder Zwiebelpulver und/oder ein oder mehrere Fischöle und gegebenenfalls

(c) weitere physiologisch verträgliche Trägerstoffe und/oder Hilfsstoffe mit Ausnahme von durchblutungsfördernden und/oder gefäßerweiternden Mitteln.

Die Erfindung betrifft außerdem die Verwendung derartiger Zusammensetzungen zur Verbesserung der Eigenschaften des Blutes bzw. zur Förderung der Durchblutung der Peripherie der Augen, des Mittelohres, des Herzens und des Cerebrums, die dadurch gekennzeichnet ist, daß man eine Gesamttagesdosis an Vitamin E von mehr als 400 mg, vorzugsweise zwischen 400 und 1800 mg und an Extrakten oder Konzentraten oder Pulvern des Knoblauchs zwischen 200 und 2000 mg bzw. an einer oder mehreren Komponenten aus der Gruppe Extrakte oder Konzentrate der Zwiebel, Fischöle zwischen 200 und 4000 mg vorsieht. Als dritte Komponente können mehrfach ungesättigte Fettsäuren und/oder deren Ester der erfindungsgemäßen Zusammensetzung beigefügt werden.

Eine der essentiellen Komponenten der erfindungsgemäßen Zusammensetzungen ist Vitamin E, das als solches oder in Form seiner physiologisch aktiven Derivate oder auch in Form einer Mischung von Vitamin E und eines der physiologisch aktiven Derivate in hoher Dosierung eingesetzt werden kann. Neben dem freien $\alpha$-Tocopherol, das die üblicherweise physiologisch aktive Form des freien Vitamin E ist, oder auch zusammen mit diesem, können also beispielsweise in bevorzugten Ausführungsformen $\alpha$-Tocopherol-Ester natürlicher oder synthetischer Herkunft verwendet werden. Darin ist die freie Hydroxygruppe des $\alpha$-Tocopherols mit physiologisch verträglichen Carbonsäuren verestert. Derartige Ester können sich beispielsweise von Essigsäure ($\alpha$-Tocopherolacetat) oder Bernsteinsäure ($\alpha$-Tocopherolsuccinat) ableiten.

Es sind jedoch auch Ester des freien $\alpha$-Tocopherols mit anderen Carbonsäuren denkbar, sofern sie physiologisch verträgliche Verbindungen sind. Das freie $\alpha$-Tocopherol kann sowohl in der D-Form als auch als DL-$\alpha$-Tocopherol eingesetzt werden.

Als zweite essentielle Komponente enthalten die erfindungsgemäßen Zusammensetzungen zur Verbesserung der Eigenschaften des Blutes eine oder mehrere Komponenten aus der Gruppe Extrakte, Konzentrate oder Pulver des Knoblauchs, Extrakte, Konzentrate oder Pulver der Zwiebel, Fischöle. Mehrfach ungesättigte Fettsäuren oder deren Ester können als dritte Komponente zugegeben werden, insbesondere bevorzugt ist dabei Lecithin.

Unter Extrakten oder Konzentraten des Knoblauchs werden dabei solche, gegebenenfalls nach bekannten Methoden herstellbaren, heute jedoch auch käuflich erhältlichen Zubereitungen des Knoblauchs verstanden, in denen die wesentlichen Wirkstoffe dieser Knolle enthalten sind. Insbesondere werden darunter im Sinne der vorliegenden Erfindung Knoblauchölmazerate, Knoblauchölextrakte oder auch Knoblauchpulver verstanden. Derartige Mazerate, Extrakte bzw. Pulver werden nach aus dem Stand der Technik bekannten Methoden hergestellt. Die Herstellverfahren bedürfen daher an dieser Stelle keiner weiteren Erläuterung. Die erfindungsgemäßen Zusammensetzungen zur Verbesserung der Eigenschaften des Blutes enthalten also neben Vitamin E in bevorzugten Ausführungsformen Knoblauchölmazerate und/oder Knoblauchölextrakte und/ oder Knoblauchpulver.

Weiter bevorzugte Ausführungsformen der erfindungsgemäßen Zusammensetzungen bestehen darin, daß diese Vitamin E in hoher Dosierung enthalten. Darunter werden solche Vitamin E-Mengen verstanden, die deutlich über den bisher in derartigen Verabreichungseinheiten enthaltenen Vitamin E-Mengen liegen und üblicherweise mindestens 200 mg Vitamin E pro Verabreichungseinheit betragen. Diese Tatsache ist eine Folge der Erkenntnis, daß die in der Vergangenheit hin und wieder eingesetzten geringen Mengen von ca. 40 mg Vitamin E pro Verabreichungseinheit wegen der zu niedrigen Dosierung völlig wirkungslos sind, da die überwiegende Menge des Vitamin E - sofern es oral verabreicht wird - durch die Magensäure zerstört wird und dadurch seine Wirksamkeit verliert. Dies wurde beispielsweise von A. Vogelsang in "Angiology 21, 275-279 (1970)" experimentell verifiziert. Nur eine Gesamttagesdosis von mindestens 400 mg Vitamin E wurde schon in der Vergangenheit als Untergrenze der wirksamen Vitamin E-Tagesdosis angesehen.

Die besonders bevorzugten Zusammensetzungen auf Knoblauch-Basis gemäß der Erfindung enthalten pro Verabreichungseinheit Vitamin E und/oder eines seiner physiologisch aktiven Derivate in Gesamtmengen von 200 bis 1200 mg, besonders bevorzugt von 300 bis 900 mg, und Knoblauchölmazerate und/oder Knoblauchölextrakte und/oder Knoblauchpulver in Gesamtmengen von 100 bis 800 mg, bevorzugt von 150 bis 500 mg.

Neben den oben genannten essentiellen Komponenten Vitamin E in hoher Dosierung und knoblauchhaltige Komponente(n) können die erfindungsgemäßen Zusammensetzungen gegebenenfalls zusätzlich noch weitere physiologisch verträgliche Trägerstoffe und/oder Hilfsstoffe enthalten. Dies können in derartigen Zusammensetzungen sonst übliche Stoffe sein. Bevorzugt werden ein oder mehrere pflanzliche Öle und/oder ein oder mehrere Emulgatoren. Deren genaue Natur wird weiter unten erläutert.

Es entspricht einer weiteren bevorzugten Ausführungsform der Erfindung, daß die Vitamin E- und die Knoblauch-Komponente(n) enthaltenden Zusammensetzungen in Form von Kapseln verabreicht werden. Es wird weiterhin erfindungsgemäß bevorzugt, daß pro Tag mindestens zwei Verabreichungseinheiten, im speziellen Fall also zwei Kapseln, einzunehmen sind, um die Mindestaufnahme an Vitamin E bzw. Knoblauchkomponente(n) sicherzustellen. Höhere Dosierungen, also die Aufnahme von mehr als zwei Verabreichungseinheiten bzw. speziell Kapseln, sind ebenfalls möglich. Dies bringt jedoch - bis auf die Aufnahme größerer Mengen und damit die Sicherstellung des therapeutischen Effektes - keine zusätzlichen Vorteile.

Es ist jedoch auch möglich, die erfindungsgemäßen Zusammensetzungen in flüssiger Form zu verabreichen. Dies kann beispielsweise in Form von Tropfen geschehen. Dabei ist sicherzustellen, daß die oben angegebenen Dosierungen eingehalten werden. Die Verabreichung von Tropfen kann besonders sinnvoll sein, wenn Patienten mit Schluckbeschwerden die Kapseln nicht einnehmen können.

Mit den erfindungsgemäßen Zusammensetzungen mit dem oben angegebenen Gehalt an Vitamin E und/oder einem seiner physiologisch aktiven Derivate und an einer oder mehreren, Knoblauchwirkstoffe enthaltenden Komponenten lassen sich die charakteristischen Blutwerte deutlich verbessern. So wird die Blutviskosität bei Einhaltung der angegebenen Mengen schon wesentlich stärker erniedrigt, als dies bei Präparaten aus dem Stand der Technik der Fall war, bei denen Knoblauch-Tagesdosierungen von 1800 mg nur eine Verbesserung der Blutviskositäts-Werte im Bereich von unter 1% erbrachten. Zudem werden die Lipid-Werte des Blutes wesentlich stärker erniedrigt, als dies mit Präparaten aus dem Stand der Technik der Fall war.

Es entspricht einer weiteren Ausführungsform der Erfindung, daß die Zusammensetzungen zur Verbesserung der Eigenschaften des Blutes Vitamin E und/oder eines seiner physiologisch aktiven Derivate in hoher Dosierung, Zwiebelöl und/oder Zwiebelölextrakte und/oder Zwiebelpulver und gegebenenfalls weitere physiologisch verträgliche Trägerstoffe und/oder Hilfsstoffe enthalten. Mit Vorteil werden die Zusammensetzung so komponiert, daß sie pro Verabreichungseinheit Vitamin E und/oder eines seiner physiologisch aktiven Derivate in Gesamtmengen von 75 bis 1200 mg, bevorzugt von 300 bis 900 mg, Zwiebelöl und/oder Zwiebelölextrakte und/oder Zwiebelpulver in Gesamtmengen von 100 bis 800 mg, bevorzugt von 150 bis 500 mg, und gegebenenfalls weitere physiologisch verträgliche Trägerstoffe und/oder Hilfsstoffe enthalten.

Eine weitere Ausführungsform der Erfindung ist darauf gerichtet, daß erfindungsgemäße Zusammensetzungen zur Verbesserung der Eigenschaften des Blutes Vitamin E und/oder eines seiner physiologisch aktiven Derivate in hoher Dosierung, ein oder mehrere Fischöle und gegebenenfalls weitere physiologisch verträgliche Trägerstoffe und/oder Hilfsstoffe enthalten. Auch hierin bedeutet der Terminus "hohe Dosierung" des Vitamin E, daß pro einzelner Verabreichungseinheit üblicherweise mindestens 200 mg Vitamin E enthalten sind, so daß - bei einer bevorzugten Verabreichung von zwei Verabreichungseinheiten pro Tag - eine Mindest-Tagesdosis an Vitamin E von 400 mg erreicht werden kann.

Als Fischöle kommen eine Vielzahl von flüssigen Fetten und Ölen in Frage, wie man sie aus marinen Lebewesen durch Auspressen, Extrahieren oder ähnliche, aus dem Stand der Technik bekannte Verfahren gewinnen kann. Typisch für derartige Fischöle ist die Kettenlängenverteilung: Es finden sich überwiegend langkettige, das heißt mehr als 16 C-Atome aufweisende Fettsäure-Reste. Ein zusätzliches charakteristisches Merkmal ist darin zu sehen, daß ein hoher Anteil ungesättigter, üblicherweise mehrfach ungesättigter Fettsäure-

Reste zu finden ist. Letztere machen mehr als 50%, bezogen auf die Gesamtzahl aller Alkylreste im jeweiligen Öl, aus.

Zusammen mit einem oder mehreren Fischölen können auch eine oder mehrere mehrfach ungesättigte Fettsäuren und/ oder deren Ester und/oder Lecithin als essentielle Komponente in den erfindungsgemäßen Zusammensetzungen enthalten sein. Zwar ist die Verwendung aller physiologisch verwertbaren, mehrfach ungesättigten Fettsäuren in den erfindungsgemäßen Zusammensetzungen möglich, es werden jedoch auch von diesen Verbindungen diejenigen bevorzugt, die im Kettenlängenbereich von $C_{16}$ im Alkylrest oder höher liegen, wobei auch hier die mehrfach ungesättigten Fettsäuren und ihre Ester bevorzugt werden. Als Ester kommen solche mit niederen Alkoholen wie Methanol oder Ethanol oder auch mit Diolen und Polyolen in Frage.

Mit Vorteil enthalten die erfindungsgemäßen Zusammensetzungen als Fischöle Lebertranöl und/oder Lachsöl, insbesondere Lachsöl mit einem hohen Anteil an Omega-3-Fettsäure. Von den mehrfach ungesättigten Fettsäuren oder deren Estern, die zusammen mit den Fischölen und Vitamin E eine erfindungsgemäße Dreierkombination bilden, sind Linolsäure und deren Ester bzw. Linolöl besonders bevorzugt.

Entsprechend einer weiteren, besonders bevorzugten Ausführungsform enthalten die erfindungsgemäßen Zusammensetzungen pro Verabreichungseinheit Vitamin E und/oder eines seiner physiologisch aktiven Derivate in Gesamtmengen von 75 bis 1200 mg, bevorzugt von 100 bis 900 mg und ein oder mehrere Fischöle und/oder mehrfach ungesättigte Fettsäuren und/oder deren Ester in Gesamtmengen von 100 bis 800 mg, bevorzugt von 200 bis 500 mg, und gegebenenfalls weitere physiologisch verträgliche Trägerstoffe und/oder Hilfsstoffe. In derartigen Zusammensetzungen ist - wie auch in den Knoblauch enthaltenden Zusammensetzungen gemäß der Erfindung - die Verwendung von Vitamin E in Form des freien $\alpha$-Tocopherols möglich. Dieses kann sowohl in der D-Form als auch in der DL-Form eingesetzt werden. Andererseits werden von der vorliegenden Erfindung ebenfalls solche Zusammensetzungen umfaßt, die Vitamin E in Form der $\alpha$-Tocopherol-Ester natürlicher oder synthetischer Herkunft enthalten. Physiologisch verträgliche Ester des $\alpha$-Tocopherols sind beispielsweise das Acetat und das Succinat. Neben diesen sind jedoch auch Ester des $\alpha$-Tocopherols mit anderen Carbonsäuren verwendbar.

Es entspricht einer bevorzugten Ausführungsform auch der Zwiebel-Komponenten bzw. Fischöle und gegebenenfalls mehrfach ungesättigte Fettsäuren oder deren Ester enthaltenden Zusammensetzungen, daß sie in Form von Kapseln verabreicht werden. Solche Kapseln bringen erhebliche Vorteile bei der Verabreichung der erfindungsgemäßen Zusammensetzungen. Es ist auch in diesem Fall zu empfehlen, daß mindestens zwei Verabreichungseinheiten, also im speziellen Fall zwei Kapseln, pro Tag verabreicht werden, die erfindungsgemäß hoch dosiertes Vitamin E und weitere Komponenten aus der Gruppe Zwiebelkomponenten und Fischöle enthalten. Auch die Dreierkombination Vitamin E, Fischöle und mehrfach ungesättigte Fettsäuren oder deren Ester können wie oben beschrieben verabreicht werden. Mit der Verabreichung von mindestens zwei Kapseln wird weitgehend sichergestellt, daß die Mindest-Tagesdosis sowohl an Vitamin E als auch an den weiteren Komponenten erreicht wird.

Es ist auch hier möglich, die erfindungsgemäßen Zusammensetzungen in flüssiger Form zu verabreichen. Dies kann beispielsweise in Form von Tropfen geschehen. Dabei ist sicherzustellen, daß die oben angegebenen Dosierungen eingehalten werden. Die Verabreichung von Tropfen kann besonders sinnvoll sein, wenn Patienten mit Schluckbeschwerden die Kapseln nicht einnehmen können.

Wie oben auch schon für die Knoblauch enthaltenden Zusammensetzungen gemäß der Erfindung angegeben, können auch die danach beschriebenen Zusammensetzungen zusätzliche, physiologisch verträgliche Trägerstoffe und/oder Hilfsstoffe enthalten. Als solche können beispielsweise ein oder mehrere pflanzliche Öle und/oder ein oder mehrere Emulgatoren genannt werden. Die pflanzlichen Öle können beispielsweise aus der Gruppe Sojaöl, Rüböl, Leinsamenöl, Sonnenblumenöl, Olivenöl oder ähnlicher, physiologisch verträglicher Öle stammen. Bevorzugt werden Rüböl und Sojaöl verwendet.

Auch als Emulgatoren kommen eine Vielzahl von Verbindungen natürlicher oder synthetischer Herkunft in Frage, die allein oder auch zu mehreren als Emulgator verwendet werden können. Bevorzugte Beispiele derartiger Emulgatoren sind Lecithin pflanzlicher (Sojalecithin) bzw. tierischer (Eilecithin) Herkunft sowie auch andere, eine Emulsion stabilisierende Phospholipide. Derartige Emulgatoren werden in Mengen von 50 bis 500 mg pro Verabreichungseinheit verwendet. Die Emulgatormenge unterliegt jedoch nur geringfügigen Beschränkungen, da es hinsichtlich der Emulgatormenge sehr auf das jeweilige zu emulgierende System ankommt. Als limitierender Faktor wird nur die Forderung angesehen, daß die Zusammensetzungen gemäß der Erfindung vollständig und möglichst feinteilig emulgiert sein müssen.

Eine beispielhafte und derzeit als bevorzugt anzusehende Zusammensetzung gemäß der Erfindung enthält pro Verabreichungseinheit Vitamin E und/oder eines seiner physiologisch aktiven Derivate in Gesamtmengen von 75 bis 300 mg, eine oder mehrere Komponente(n) aus der Gruppe Zwiebelöl, Fischöl, Lachsöl und Lebertranöl in Gesamtmengen von 200 bis 500 mg und ein oder mehrere pflanzliche Öle und/oder Lecithin natürlicher oder synthetischer Herkunft. Wenn auch für die genannten Zusammensetzungen die Kapselform vorteilhafter und damit auch bevorzugt ist, ist es doch möglich, auch andere Verabreichungsformen zuzubereiten. Beispielsweise sind Dragees oder Tabletten zu nennen, die den Vorteil hätten, daß dann sowohl das Vitamin E als auch die weiteren essentiellen Komponenten in Pulver- bzw.

fester Form verwendet werden können. Auch bei der Herstellung derartiger Zubereitungen werden die üblichen Hilfsstoffe und Träger verwendet. Zusätze von geringen Mengen der oben genannten Emulgatoren begünstigen die Resorption und Verarbeitung.

Die Herstellung der erfindungsgemäßen Zusammensetzungen erfolgt in aus dem Stand der Technik bekannter Weise. Diese kann darin bestehen, daß die einzelnen Komponenten, sofern sie pulverartige Konsistenz haben, in üblichen Pulvermischern miteinander vermischt werden und anschließend mit Hilfe der oben genannten Trägerstoffe bzw. Hilfsstoffe in die Form gebracht werden, in der man sie verabreichen möchte. Beispielsweise können die entsprechenden pulverförmigen Komponenten dann mit Ölen und Emulgatoren vermischt werden und die entstehenden ölig-zähen Flüssigkeiten in Kapseln üblicher Zusammensetzung eingebracht werden. Es ist jedoch auch möglich, aus festen bzw. pulverförmigen Komponenten-Mischungen mit Hilfe üblicher Trägerstoffe oder Hilfsstoffe Tabletten zu pressen und diese - sofern erwünscht - mit einer üblichen Dragierschicht zu versehen.

Entsprechend erfolgt die Verarbeitung öliger Komponenten dadurch, daß Vitamin E bzw. seine Derivate mit den öligen Komponenten innig vermischt werden und diese dann in übliche Kapseln eingebracht werden. Dadurch werden gut handhabbare und eine bestimmte Dosis aller Komponenten enthaltende Verabreichungseinheiten hergestellt, die es ermöglichen, genau zu überwachen bzw. anzugeben, wieviel einzelne Verabreichungseinheiten eingenommen werden müssen, um die Mindest-Tagesdosis der oben näher beschriebenen Wirkstoffe zu erreichen.

Von der Erfindung umfaßt ist ebenfalls die Verwendung der oben näher beschriebenen, Knoblauch enthaltenden Zusammensetzungen zur Verbesserung der Eigenschaften des Blutes bzw. zur Förderung der Durchblutung der Peripherie der Augen, des Mittelohres, des Herzens und des Cerebrums. Bei der Verwendung der genannten Zusammensetzungen wird erfindungsgemäß eine Gesamttagesdosis an Vitamin E zwischen 400 und 1800 mg, bevorzugt zwischen 500 und 1500 mg, und an Extrakten oder Konzentraten oder Pulvern des Knoblauchs zwischen 200 und 2000 mg, bevorzugt zwischen 300 und 1000 mg, vorgesehen.

Von der Erfindung ebenfalls umfaßt ist die Verwendung der Zusammensetzungen, die Zwiebeln enthaltende Komponenten und/oder Fischöl(e) enthalten, zur Verbesserung der Eigenschaften des Blutes bzw. zur Förderung der Durchblutung der Peripherie der Augen, des Mittelohres, des Herzens und des Cerebrums. Als dritte Komponente können auch mehrfach ungesättigte Fettsäuren und/oder deren Ester der Zusammensetzung beigefügt werden. Bei der Verwendung derartiger Zusammensetzungen wird eine Gesamttagesdosis an Vitamin E von mindestens 400 mg, bevorzugt zwischen 500 und 1500 mg, besonders bevorzugt zwischen 600 und 1000 mg, und an einer oder mehreren Komponenten aus der Gruppe Extrakte oder Konzentrate der Zwiebel, Fischöle und mehrfach ungesättigte Fettsäuren oder deren Ester zwischen 200 und 4000 mg, bevorzugt zwischen 300 und 2000 mg, vorgesehen. Eine höhere Dosierung ist selbstverständlich möglich.

Besonders bevorzugt ist die Verwendung der genannten Zusammensetzungen zur Senkung der Lipidwerte des Blutes und/oder zur Senkung des Blutdrucks. Die überraschende synergistische Wirkung der Vitamin E-Komponente(n) einerseits und der Komponente(n) andererseits aus der Gruppe Knoblauch, Zwiebeln enthaltende Komponenten oder Fischöle führt dazu, daß die Mengen an Einzelkomponenten deutlich reduziert werden können, ohne daß ein Wirkungsverlust in irgendeiner Weise auftritt. Vielmehr werden bei den angegebenen Dosierungen deutliche Verbesserungen der charakteristischen Eigenschaften des Blutes, insbesondere der Erythrozytenflexibilität, sowie auch eine deutliche Absenkung der Lipidwerte bzw. Blutdruckwerte des Blutes beobachtet.

Ein ebenfalls überraschendes Ergebnis der vorliegenden Erfindung, das heißt der Verwendung der oben genannten Zusammensetzungen, ist darin zu sehen, daß die Zugabe von Lecithin als Emulgator zu den beiden anderen Wirkstoffen, das heißt zu der Vitamin E-Komponente und der Knoblauch-, Zwiebel-, Fischöl- oder Fett-Komponente, die charakteristischen Eigenschaften des Blutes noch günstiger beeinflußt. Dabei werden besonders vorteilhafte Ergebnisse erzielt, wenn man Lecithin-Präparate mit einem erhöhten Phospholipid-Gehalt, verglichen mit natürlichen, pflanzlichen oder tierischen Phospholipiden, verwendet.

Die Erfindung wird durch die nachfolgenden Beispiele näher erläutert, wobei Rezepturen für einzelne Zusammensetzungen gemäß der Erfindung angegeben werden.

**Beispiel 1**

Es wurden Kapseln hergestellt, die folgende Komponenten enthielten:
370 mg Knoblauchölmazerat auf Basis Rüböl (Volumenverhältnis 1:1);
400 mg DL-$\alpha$-Tocopherolacetat.

Es wird empfohlen, zweimal täglich eine Kapsel (über den Tag verteilt) einzunehmen.

7

**Beispiel 2**

Es wurden Kapseln hergestellt, die folgende Komponenten enthielten:
200 mg Knoblauchölmazerat auf Basis Rüböl (Volumenverhältnis 1:1);
200 mg DL-$\alpha$-Tocopherolacetat;
250 mg Sojalecithin (Phospholipidgehalt ca. 60%).
Es wird empfohlen, zweimal zwei Kapseln über den Tag verteilt einzunehmen.

**Beispiel 3**

Es wurden Kapseln hergestellt, die folgende Komponenten enthielten:
300 mg Knoblauchölmazerat auf Basis Rüböl (Volumenverhältnis 1:1);
400 mg D-$\alpha$-Tocopherol-Konzentrat.

**Beispiel 4**

Es wurden Kapseln hergestellt, die folgende Komponenten enthielten:
200 mg Knoblauchölmazerat auf Basis Rüböl (Volumenverhältnis 1:1);
200 mg D-$\alpha$-Tocopherol-Konzentrat (1000 i.u./1 g);
200 mg Sojalecithin (Phospholipidgehalt ca. 54%).
Es wird empfohlen, zweimal zwei Kapseln über den Tag verteilt einzunehmen.

**Beispiel 5**

Es wurden Kapseln hergestellt, die folgende Komponenten enthielten:
150 mg Bulbus alli sativi sicc. (Erg 8);
200 mg DL-$\alpha$-Tocopherolacetat;
100 mg Rüböl oder Sojaöl.
Es wird empfohlen, zweimal zwei Kapseln über den Tag verteilt einzunehmen.

**Beispiel 6**

Es wurden Dragees hergestellt, die folgende Komponenten enthielten:
100 mg Bulbus alli sativi sicc. (Erg B6);
120 mg Vitamin E-Succinat.
Es wird empfohlen, dreimal zwei Dragees über den Tag verteilt einzunehmen.

**Beispiel 7**

Es wurden Kapseln hergestellt, die folgende Komponenten enthielten:
400 mg Fischöl;
135 mg Vitamin E DL-$\alpha$-Tocopherolacetat.
Es wird empfohlen, dreimal zwei Kapseln täglich einzunehmen.

**Beispiel 8**

Es wurden Kapseln hergestellt, die folgende Komponenten enthielten:
400 mg Fischöl;
200 mg Vitamin E;
50 mg Sojaöl.
Es wird empfohlen, zweimal zwei Kapseln täglich einzunehmen.

**Beispiel 9**

Es wurden Kapseln hergestellt, die folgende Komponenten enthielten:
400 mg Lachsöl;
135 mg Vitamin E D-$\alpha$-Tocopherol-Konzentrat;
50 mg Rüböl.

Es wird empfohlen, dreimal zwei Kapseln täglich einzunehmen.

**Beispiel 10**

Es wurden Kapseln hergestellt, die folgende Komponenten enthielten:
500 mg Lebertranöl (Oleum jecoris);
400 mg DL-$\alpha$-Tocopherolacetat;
70 mg Sojaöl.
Es wird empfohlen, zweimal täglich eine Kapsel einzunehmen.

**Beispiel 11**

Es wurden Kapseln hergestellt, die folgende Komponenten enthielten:
300 mg Lebertranöl (Oleum jecoris);
200 mg D-$\alpha$-Tocopherol-Konzentrat;
150 mg Sojaöl.
Es wird empfohlen, zweimal zwei Kapseln täglich einzunehmen.

**Beispiel 12**

Es wurden Kapseln hergestellt, die folgende Komponenten enthielten:
400 mg Lachsöl mit hohem Anteil an Omega-3-Fettsäure;
135 mg Vitamin E;
6 mg Tween 80.
Es wird empfohlen, dreimal zwei Kapseln täglich einzunehmen.

**Beispiel 13**

Es wurden Kapseln hergestellt, die folgende Komponenten enthielten:
250 mg Zwiebelöl;
200 mg Vitamin E;
50 mg Sojaöl.
Es wird empfohlen, zweimal zwei Kapseln täglich einzunehmen.

**Beispiel 14**

Es wurden Kapseln hergestellt, die folgende Komponenten enthielten:
400 mg Linolöl;
135 mg Vitamin E;
65 mg Sojaöl.
Es wird empfohlen, dreimal zwei Kapseln täglich einzunehmen.

**Beispiel 15**

Es wurden Kapseln hergestellt, die folgende Komponenten enthielten:
300 mg Lachsöl;
75 mg Vitamin E.
Es wird empfohlen, dreimal drei Kapseln täglich einzunehmen.

**Beispiel 16**

Es wurden Kapseln hergestellt, die folgende Komponenten enthielten:
400 mg Lachsöl;
75 mg Vitamin E.
Es wird empfohlen, dreimal drei Kapseln täglich einzunehmen.

**Beispiel 17**

Es wurden Kapseln hergestellt, die folgende Komponenten enthielten:
200 mg Lebertranöl (Oleum jecoris);
75 mg Vitamin E.
Es wird empfohlen, dreimal drei Kapseln täglich einzunehmen.

**Beispiel 18**

Es wurden Kapseln hergestellt, die folgende Komponenten enthielten:
400 mg Linolöl;
75 mg Vitamin E.
Es wird empfohlen, dreimal drei Kapseln täglich einzunehmen.

**Beispiel 19**

Voruntersuchungen mit hochdosiertem Vitamin E hatten ergeben, daß durch diese Substanz eine signifikante Zunahme der Erythrozyten-Flexibilität bei Gesunden induziert wird. Es sollte nun geprüft werden, in welchem Maße dies auch durch eine kombinierte Therapie von Vitamin E und Knoblauchölmazerat (1:1) zu erreichen sei. Dazu wurden zehn freiwillige männliche Probanden im Durchschnittsalter von 30 (23 bis 39) Jahren untersucht, die folgenden Kriterien entsprachen: keine klinisch-anamnestischen Zeichen von Krankheit, normales Routinelabor, keine Einnahme von weiteren Medikamenten vor oder während der Studie, Konstanthaltung möglichst aller Lebensgewohnheiten während des Untersuchungszeitraumes.

Blutentnahmen erfolgten vor Therapiebeginn und nach zweiwöchiger oraler Medikation von zweimal einer Kapsel mit 400 mg Vitamin E und 370 mg Knoblauchölmazerat (1:1) täglich zu den Mahlzeiten. Als Vergleich wurde der gleichen Gruppe später dreimal eine Kapsel "Knoblauch Sanhelios", 270 mg-Kapseln (1:1), zu den Mahlzeiten gegeben.

Folgende Parameter wurden bestimmt:

1. Blutviskosität bei 37°C im LS 30 (Contraves) Rotationsviskometer mit D 95; 2,4; 0,7; $s^{-1}$ (1)
   a) nativ
   b) bei einem standardisierten Hämatokrit von 45%
2. Plasmaviskosität im Kapillarviskometer bei 37°C (2)
3. Erythrozyten-Flexibilität in einem Filtrationssystem (3)
4. Erythrozyten-Aggregation in einem Aggregometer (Myrenne) (4)
5. Hämatokrit mit einer Mikrohämatokrit-Zentrifuge (Heraeus Christ)
6. kleines Blutbild (TOA Counter)
7. Blutkörperchensenkungsgeschwindigkeit (BKS) nach Westergren
8. Triglyceride
9. Gesamtcholesterol
10. LDL

Es wurde Blut wie folgt entnommen: Der Proband lag mindestens 10 Minuten. Der Oberarm wurde mit 40 mmHg gestaut, mit einer 20 G Nadel wurde eine Vena cubitalis punktiert, der Stau wurde gelöst und ca 1 Minute lang abgewartet, dann wurde Blut unter sanftem Zug entnommen und sogleich mit Heparin (12,5 U/ml) antikoaguliert.

Die statistische Auswertung erfolgte nach dem nichtparametrischen Verfahren von Wilcoxon und Wilcox für gepaarte Stichproben.

Ergebnisse:

A.0 : Knoblauchölmazerat 1:1 (Sanhelios), 270 mg-Kapseln, dreimal eine Kapsel täglich zu den Mahlzeiten. Die Ergebnisse sind in Tabelle 1 aufgeführt.

A.1 : Hämorheologie: Keine Veränderung

A.2 : Lipidstoffwechsel: Alle drei gemessenen Parameter zeigen eine Senkung, davon sind Triglyzeride und LDH statistisch signifikant.

B.0 : Vitamin E, DL-$\alpha$-Tocopherolacetat 400 mg + 370 mg Knoblauchölmazerat 1:1, zweimal eine Kapsel täglich zu den Mahlzeiten. Tabelle 2 zeigt die Ergebnisse.

B.1 : Hämorheologie: Eine Senkung von Blutviskosität, gemessen bei Standard-Hämatokrit, Plasmaviskositat und Erythrozyten-Flexibilität ist zu beobachten, davon sind die Senkung der Blutviskosität und die Verbesserung der Erythrozyten-Flexibilität statistisch signifikant.

B.2 : Lipidstoffwechsel: Alle drei gemessenen Parameter (Triglyzeride, Gesamtcholesterin und LDL-Cholesterin) zeigen eine Senkung, was bei LDL das Signifikantniveau erreicht.

Es ist zu bemerken, daß alle drei Parameter des Lipidstoffwechsels bei beiden Versuchen eine Senkung zeigen. Bei den Versuchen mit Knoblauchölmazerat allein fällt die Senkung der Triglyzeride signifikant aus; die Ausgangswerte waren hier höher als bei der Kombination.

Die Werte der Triglyzeride bei

```
Knoblauch allein   141 ± 34   nach 14 Tagen   126 ± 30
                              3 x 1 Kapsel

Knoblauch +·       133 ± 36   nach 14 Tagen   126 ± 33
Vitamin E                     2 x 1 Kapsel
```

Die Endwerte nach der Einnahme sind bei beiden Versuchen identisch.

Ergebnis:

Die Verbesserung der normalen Erythrozyten-Flexibilität und Blutviskosität ist als Verbesserung der Blutfluidität zu deuten. Insbesondere in der Mikrozirkulation könnte diesem Befund Bedeutung zukommen. Es ist denkbar, daß bei pathologischen Ausgangswerten dieser Effekt quantitativ deutlicher ausfällt. Durch die Einnahme von Vitamin E allein (2 x 400 mg täglich) wurde in einer früheren Arbeit gezeigt, daß eine signifikante Verbesserung der Erythrozyten-Flexibilität stattgefunden hat. Der Zusatz von Knoblauchölmazerat zu Vitamin E zeigt zusätzlich eine signifikante Senkung der Blutviskosität. Eine zusätzliche Verbesserung der Blutfluidität durch die Einnahme der Kombination gegenüber Vitamin E allein ist daher zu erwarten.

Die lipidsenkende Wirkung von Knoblauch wurde durch den Zusatz von Vitamin E nicht negativ beeinträchtigt.

Tabelle 1

| P A R A M E T E R | BASELINE | NACH 2 WOCHEN |
|---|---|---|
| Vollblut       0,7 1/s | 21,7±4,2 | 21,3±4,2 |
| Viskosität   2,4 1/s | 13,2±2,4 | 13,1±1,6 |
| in mPas      94,5 1/s | 4,97±0,57 | 4,93±0,43 |
| Blutvisk.     0,7 1/s | 22,8±2,9 | 22,2±2,5 |
| bei 45% Hk   2,4 1/s | 13,7±0,8 | 13,6±1,2 |
| in mPas      94,5 1/s | 5,1±0,3 | 5,1±0,4 |
| Plasma Visk. (mPas) | 1,23±0,07 | 1,22±0,10 |
| Ery. Flexibilität | 55,3±8.5 | 55,0±8.6 |
| Ery. Aggregation | 10,8±2,8 | 10,8±2,7 |
| Plasma C.O.D.(mmHg) | 25,8±1,4 | 24,9±1,4 |
| Leukozyten ($10^3$/µl) | 5,8±1.0 | 5,9±0.9 |
| Hämatokrit    (%) | 44,1±3,5 | 43,7±2,4 |
| Hämoglobin    (g/l) | 162±14 | 161±12 |
| Erythrozyten ($10^6$/µl) | 5,06±0,42 | 5,15±0,40 |
| Triglyceride (mg/dl) | 141±34 | 126±30 *** |
| Cholesterin  (mg/dl) | 143±28 | 136±22 |
| LDL          (mg/dl) | 114±22 | 97±17  * |
| RR syst.      (mmHg) | 128±11 | 128±14 |
| RR diast.     (mmHg) | 80±5 | 81±7 |

Student's t-Test:  *=p<0,05;  **=p<0,01;  ***=p<0,001

## Tabelle 2

| PARAMETER | | BASELINE | NACH 2 WOCHEN |
|---|---|---|---|
| Vollblut | 0,7 l/s | 23,6±3,6 | 20,6±5,2 |
| viskosität | 2,4 l/s | 12,7±1,5 | 12,2±2,4 |
| in mPas | 94,5 l/s | 4,9±0,4 | 4,7±0,6 |
| Blutvisk. | 0,7 l/s | 26,1±1,4 | 22,9±2,6 ** |
| bei 45% Hk | 2,4 l/s | 13,8±0,8 | 13,6±0,8 |
| in mPas | 94,5 l/s | 5,1±0,3 | 5,1±0,3 |
| Plasma visk. (mPas) | | 1,24±0,07 | 1,22±0,07 |
| Ery. Flexibilität | | 55,5±4.8 | 57,3±4.8 * |
| Ery. Aggregation | | 10,6±3,4 | 10,4±3,7 |
| Plasma C.O.D.(mmHg) | | 24,8±1,0 | 25,1±1,2 |
| Leukozyten ($10^3$/ul) | | 5,5±1,0 | 5,4±1,0 |
| Hämatokrit (%) | | 43,4±2,5 | 42,4±3,3 |
| Hämoglobin (g/l) | | 156± 9 | 157±13 |
| Erythrozyten ($10^6$/ul) | | 5,01±0,42 | 4,96±0,45 |
| Triglyceride (mg/dl) | | 133±36 | 126±33 |
| Cholesterin (mg/dl) | | 141±23 | 131±29 |
| LDL (mg/dl) | | 108±19 | 91±25 * |
| RR syst. (mmHg) | | 136±8 | 135±10 |
| RR diast. (mmHg) | | 80±6 | 81±7 |

Student t-Test: *=p<0,05; **=p<0,01; ***=p<0,001

## Patentansprüche

1. Zusammensetzungen zur Verbesserung der Eigenschaften des Blutes, enthaltend pro Verabreichungs-einheit

EP 0 273 407 B1

(a) Vitamin E und/oder eines seiner physiologisch aktiven Derivate in hoher Dosierung von 75 bis 1200 mg,

(b) eine oder mehrere Komponente(n) aus der Gruppe Extrakte oder Konzentrate des Knoblauchs, Knoblauchölmazerate und/oder Knoblauchölextrakte und/oder Knoblauchpulver, Extrakte oder Konzentrate der Zwiebel oder Zwiebelöl und/oder Zwiebelölextrakte und/oder Zwiebelpulver und/oder ein oder mehrere Fischöle und gegebenenfalls

(c) weitere physiologisch verträgliche Trägerstoffe und/oder Hilfsstoffe mit Ausnahme von durchblutungsfördernden und/oder gefäßerweiternden Mitteln.

2. Zusammensetzung nach Anspruch 1, enthaltend neben den Komponenten (a) und (b) mehrfach ungesättigte Fettsäuren und/oder deren Ester und/oder Lecithin als dritte Komponente.

3. Zusammensetzungen nach Anspruch 1 oder 2, enthaltend pro Verabreichungseinheit

(a) Vitamin E und/oder eines seiner physiologisch aktiven Derivate in Gesamtmengen 200 bis 900 mg,

(b) Knoblauchölmazerate und/oder Knoblauchölextrakte und/oder Knoblauchpulver in Gesamtmengen von 100 bis 800 mg, bevorzugt von 150 bis 500 mg.

4. Zusammensetzungen nach Anspruch 1, enthaltend pro Verabreichungseinheit

(a) Vitamin E und/oder eines seiner physiologisch aktiven Derivate in Gesamtmengen bevorzugt von 300 bis 900 mg,

(b) Zwiebelöl und/oder Zwiebelölextrakte und/oder Zwiebelpulver in Gesamtmengen von 100 bis 800 mg, bevorzugt von 150 bis 500 mg.

5. Zusammensetzungen nach Anspruch 1, enthaltend als Fischöle Lebertranöl und/oder Lachsöl, insbesondere mit einem hohen Anteil an Omega-3-Fettsäure.

6. Zusammensetzungen nach Anspruch 2, enthaltend als mehrfach ungesättigte Fettsäuren oder deren Ester Linolsäure und/oder Linolöl.

7. Zusammensetzungen nach Ansprüchen 1 bis 6, enthaltend pro Verabreichungseinheit

(a) Vitamin E und/oder eines seiner physiologisch aktiven Derivate in Gesamtmengen von 75 bis 1200 mg, bevorzugt von 100 bis 900 mg,

(b) ein oder mehrere Fischöle in Gesamtmengen von 100 bis 800 mg, bevorzugt von 200 bis 500 mg.

8. Zusammensetzungen nach Ansprüchen 1 bis 7, enthaltend Vitamin E in Form des freien $\alpha$-Tocopherols oder in Form der $\alpha$-Tocopherol-Ester natürlicher oder synthetischer Herkunft.

9. Zusammensetzungen nach Ansprüchen 1 bis 8, dadurch gekennzeichnet, daß sie in Form von Kapseln oder in flüssiger Form verabreicht werden.

10. Zusammensetzungen nach Ansprüchen 1 bis 9, enthaltend als physiologisch verträgliche Trägerstoffe und/oder Hilfsstoffe ein oder mehrere pflanzliche Öle und/oder ein oder mehrere Emulgatoren.

11. Zusammensetzungen nach Ansprüchen 2 bis 10, enthaltend pro Verabreichungseinheit

(a) Vitamin E und/oder eines seiner physiologisch aktiven Derivate in Gesamtmengen von 75 bis 300 mg,

(b) eine oder mehrere Komponente(n) aus der Gruppe Zwiebelöl, Fischöl, Lachsöl und Lebertranöl in Gesamtmengen von 200 bis 500 mg und

(c) ein oder mehrere pflanzliche(s) Öl(e) und/oder Lecithin natürlicher oder synthetischer Herkunft.

12. Verwendung der Zusammensetzungen nach Ansprüchen 1 bis 11 zur Herstellung eines Mittels zur Verbesserung der Eigenschaften des Blutes bzw. zur Förderung der Durchblutung der Peripherie der Augen, des Mittelohres, des Herzens und des Cerebrums.

13. Verwendung der Zusammensetzungen nach Ansprüchen 1 bis 11 zur Herstellung eines Mittels zur Senkung der Lipidwerte des Blutes und/oder zur Senkung des Blutdrucks.

14

**Claims**

1. Compositions for improving the properties of blood, containing per administration unit
   (a) vitamin E and/or one of its physiologically active derivatives in high dosage of from 75 to 1,200 mg,
   (b) one or more components from the group of extracts or concentrates of garlic, garlic oil macerations and/or garlic oil extracts and/or garlic powders, extracts or concentrates of onion or onion oil and/or onion oil extracts and/or onion powders, and/or one or more fish oils, and optionally
   (c) further physiologically compatible carrier substances and/or adjuvants, with the exception of blood flow stimulants and/or vasodilators.

2. The composition according to claim 1, in addition to components (a) and (b) containing polyunsaturated fatty acids and/or esters thereof and/or lecithin as the third component.

3. Compositions according to claim 1 or 2, containing per administration unit
   (a) vitamin E and/or one of its physiologically active derivatives in total amounts of from 200 to 900 mg,
   (b) garlic oil macerations and/or garlic oil extracts and/or garlic powders in total amounts of from 100 to 800 mg, preferably from 150 to 500 mg.

4. Compositions according to claim 1, containing per administration unit
   (a) vitamin E and/or one of its physiologically active derivatives in total amounts of from 300 to 900 mg,
   (b) onion oil and/or onion oil extracts and/or onion powders in total amounts of from 100 to 800 mg, preferably from 150 to 500 mg.

5. Compositions according to claim 1, as the fish oil containing cod-liver oil and/or salmon oil, particularly having a high proportion of $\omega$-3-fatty acid.

6. Compositions according to claim 2, as the polyunsaturated fatty acids and/or esters thereof containing linoleic acid and/or linoleic oil.

7. Compositions according to claims 1 to 6, containing per administration unit
   (a) vitamin E and/or one of its physiologically active derivatives in total amounts of from 75 to 1,200 mg, preferably from 100 to 900 mg,
   (b) one or more fish oils in total amounts of from 100 to 800 mg, preferably from 200 to 500 mg.

8. Compositions according to claims 1 to 7, containing vitamin E in the form of free $\alpha$-tocopherol or in the form of $\alpha$-tocopherol esters of natural or synthetic origin.

9. Compositions according to claims 1 to 8, characterized in that they are administered in the form of capsules or in liquid form.

10. Compositions according to claims 1 to 9, as the physiologically compatible carrier substances and/or adjuvants containing one or more vegetable oils and/or one or more emulsifiers.

11. Compositions according to claims 2 to 10, containing per administration unit
    (a) vitamin E and/or one of its physiologically active derivatives in total amounts of from 75 to 300 mg,
    (b) one or more components from the group of onion oil, fish oil, salmon oil, and cod-liver oil in total amounts of from 200 to 500 mg, and
    (c) one or more vegetable oils and/or lecithin of natural or synthetic origin.

12. Use of the compositions according to claims 1 to 11 for preparing an agent to improve the properties of blood and/or stimulate blood flow in the periphery of the eyes, the middle ear, the heart, and the cerebrum.

**13.** Use of the compositions according to claims 1 to 11 for preparing an agent to reduce the blood lipid values and/or reduce blood pressure.

**Revendications**

**1.** Composés visant à améliorer les propriétés du sang, comprenant par unité d'administration:
(a) de la vitamine E et/ou l'un de ses dérivés biologiquement actifs selon un dosage élevé de 75 à 1200 mg,
(b) un ou plusieurs composants du groupe suivant: extraits ou concentrés d'ail, macérats et/ou extraits d'essence d'ail, et/ou ail en poudre, extraits ou concentrés d'oignon ou essence d'oignon et/ou extraits d'essence d'oignon et/ou oignon en poudre et/ou une ou plusieurs huiles de poisson, et le cas échéant,
(c) des matières support biologiquement tolérés et/ou des agents auxiliaires à l'exception des anti-ischémiques et/ou des vasodilatateurs.

**2.** Composé selon la revendication 1, comprenant comme troisième composant, outre les composants (a) et (b), des acides gras poly-insaturés et/ou leurs esters et/ou de la lécithine.

**3.** Composés seion la revendication 1 ou 2, comprenant par unité d'administration :
(a) de la vitamine E et/ou un de ses dérivés physiologiquement actifs à raison de 200 à 900 mg au total,
(b) des macérats et/ou des extraits d'essence d'ail et/ou de l'ail en poudre à raison de 100 à 800 mg au total - et de préférence de 150 à 500 mg.

**4.** Composés selon la revendication 1, comprenant par unité:
(a) de la vitamine E et/ou l'un de ses dérivés physiologiquement actifs de préférence à raison de 300 à 900 mg au total,
(b) de l'essence d'oignon et/ou des extraits d'essence d'oignon et/ou de l'oignon en poudre à raison de 100 à 800 mg au total - et de préférence de 150 à 500 mg.

**5.** Composés selon la revendication 1, comprenant, comme huiles de poisson, de l'huile de foie de poisson et/ou de l'huile de saumon - et en particulier une huile contenant une proportion importante d'acides gras oméga-3.

**6.** Composés selon la revendication 2, comprenant de l'acide linoléique et/ou de l'huile linoléique comme acides gras poly-insaturés.

**7.** Composés selon les revendications 1 à 6, comprenant par unité d'administration :
(a) de la vitamine E et/ou l'un de ses dérivés physiologiquement actifs à raison de 75 à 1200 mg au total - et de préférence de 100 à 900 mg
(b) une ou plusieurs huiles de poisson à raison de 100 à 800 mg au total - et de préférence de 200 à 500 mg.

**8.** Composés selon les revendications 1 à 7, comprenant de la vitamine E sous forme d'$\alpha$-tocophérol libre ou d'ester d'$\alpha$-tocophérol d'origine naturelle ou synthétique.

**9.** Composés selon les revendications 1 à 8, caractérisés en ce qu'ils sont administrés sous forme de capsules ou sous forme liquide.

**10.** Composés selon les revendications 1 à 9, comprenant, comme transporteurs physiologiquement tolérés et/ou agents auxiliaires, une ou plusieurs huiles végétales et/ou un ou plusieurs émulsifiants.

**11.** Composés selon les revendications 2 à 10, comprenant par unité:
(a) de la vitamine E et/ou l'un de ses dérivés physiologiquement actifs à raison de 75 à 300 mg au total,
(b) un ou plusieurs composants du groupe suivant: essence d'oignon, huile de poisson, huile de saumon, huile de foie de poisson à raison de 200 à 500 mg au total, et
(c) une ou plusieurs huiles végétales et/ou de la lécithine d'origine naturelle ou synthétique.

**12.** Utilisation de composés selon les revendications 1 à 11 pour préparer une substances visant à améliorer les propriétés sanguines et/ou à action anti-ischémique dans la zone péri-oculaire, l'oreille moyenne, le coeur et le cerveau.

**13.** Utilisation des composés selon les revendications 1 à 11 pour préparer une substance visant à abaisser la teneur du sang en lipides et/ou la pression sanguine.